# EUROPEAN PATENT APPLICATION

(11) **EP 2 232 985 A2**
(43) Date of publication of application: **29.09.2010**
(21) Application number: 09700180.4
(22) Date of filing: 05.01.2009
(51) Int. Cl.: A01K 67/027

(54) **METHOD FOR PRODUCING CLONED TRANSGENIC CANIDAE**

(30) Priority: 04.01.2008 KR 20080001106
(71) Applicant: SEOUL NATIONAL UNIVERSITY INDUSTRY FOUNDATION, Gwanak-gu Seoul 151-050 (KR)
(72) Inventor: LEE, Byeong Chun, Seoul 151-742 (KR); KIM, Min Kyu, Seoul 151-742 (KR); JANG, Goo, Seoul 151-742 (KR); OH, Hyun Ju, Seoul 151-742 (KR); HONG, So Gun, Seoul 151-742 (KR); PARK, Jung Eun, Seoul 151-742 (KR); KANG, Jung Taek, Seoul 151-742 (KR); RA, Jeong Chan, Suwon-si Gyeonggi-do 440-330 (KR); KIM, Te Oan, Daegu 706-777 (KR)
(74) Representative: Hiebl, Inge Elisabeth
(86) International application number: PCT/KR2009/000028
(87) International publication number: WO 2009/088189

(57) **Abstract**

The present invention relates to a method for producing a cloned transgenic canine, and more particularly to a method for producing a cloned transgenic canine containing a target gene, the method comprising: enucleating a canine oocyte to prepare an enucleated canine oocyte; transferring a cell transfected with the target gene into the canine enucleated oocyte to generate a nuclear transfer embryo; and transferring the nuclear transfer embryo into a surrogate mother. The present invention demonstrates the possibility of producing large amounts of disease model animals by the successful introduction of a foreign gene and is useful for research purposes in the veterinary science, anthropology and medical science fields.

<110>    Seoul National University Industry Foundation

<120>    Method For Producing Cloned Transgenic Dog

<130>    PP-B0679

<150>    KR10-2008-0001106
<151>    2008-01-04

<160>    7

<170>    KopatentIn 1.71

<210>    1
<211>    5678
<212>    DNA
<213>    Artificial Sequence

<220>
<223>    plasmid


<400>    1
tttgaaagac cccacccgta ggtggcaagc tagcttaagt aacgccactt tgcaaggcat        60

ggaaaaatac ataactgaga atagaaaagt tcagatcaag gtcaggaaca aagaaacagc       120

tgaataccaa acaggatatc tgtggtaagc ggttcctgcc ccggctcagg gccaagaaca       180

gatgagacag ctgagtgatg ggccaaacag gatatctgtg gtaagcagtt cctgccccgg       240

ctcggggcca agaacagatg gtccccagat gcggtccagc cctcagcagt ttctagtgaa       300

tcatcagatg tttccagggt gccccaagga cctgaaaatg accctgtacc ttatttgaac       360

taaccaatca gttcgcttct cgcttctgtt cgcgcgcttc cgctctccga gctcaataaa       420

agagcccaca acccctcact cggcgcgcca gtcttccgat agactgcgtc gcccgggtac       480

ccgtattccc aataaagcct cttgctgttt gcatccgaat cgtggtctcg ctgttccttg       540

ggagggtctc ctctgagtga ttgactaccc acgacggggg tctttcattt gggggctcgt       600

ccgggatttg gagacccctg cccagggacc accgacccac caccgggagg taagctggcc       660

agcaacttat ctgtgtctgt ccgattgtct agtgtctatg tttgatgtta tgcgcctgcg       720

tctgtactag ttagctaact agctctgtat ctggcggacc cgtggtggaa ctgacgagtt       780

ctgaacaccc ggccgcaacc ctgggagacg tcccagggac tttgggggcc gtttttgtgg       840

cccgacctga ggaagggagt cgatgtggaa tccgaccccg tcaggatatg tggttctggt       900

aggagacgag aacctaaaac agttcccgcc tccgtctgaa tttttgcttt cggtttggaa       960

ccgaagccgc gcgtcttgtc tgctgcagcg ctgcagcatc gttctgtgtt gtctctgtct      1020

gactgtgttt ctgtatttgt ctgaaaatta gggccagact gttaccactc ccttaagttt      1080

gaccttaggt cactggaaag atgtcgagcg gatcgctcac aaccagtcgg tagatgtcaa      1140

gaagagacgt tgggttacct tctgctctgc agaatggcca acctttaacg tcggatggcc 1200

gcgagacggc acctttaacc gagacctcat cacccaggtt aagatcaagg tcttttcacc 1260

tggcccgcat ggacacccag accaggtccc ctacatcgtg acctgggaag ccttggcttt 1320

tgacccccct ccctgggtca agccctttgt acaccctaag cctccgcctc ctcttcctcc 1380

atccgccccg tctctccccc ttgaacctcc tcgttcgacc ccgcctcgat cctcccttta 1440

tccagccctc actccttctc taggcgccgg aattccgatc tgatagcttg ccacaacccg 1500

taccaaagat ggatagatcc ggaaagcctg aactcaccgc gacgtctgtc gagaagtttc 1560

tgatcgaaaa gttcgacagc gtctccgacc tgatgcagct ctcggagggc gaagaatctc 1620

gtgctttcag cttcgatgta ggagggcgtg gatatgtcct gcgggtaaat agctgcgccg 1680

atggtttcta caaagatcgt tatgtttatc ggcactttgc atcggccgcg ctcccgattc 1740

cggaagtgct tgacattggg gaattcagcg agagcctgac ctattgcatc tcccgccgtg 1800

cacagggtgt cacgttgcaa gacctgcctg aaaccgaact gcccgctgtt ctgcagccgg 1860

tcgcggaggc catggatgcg atcgctgcgg ccgatcttag ccagacgagc gggttcggcc 1920

cattcggacc gcaaggaatc ggtcaataca ctacatggcg tgatttcata tgcgcgattg 1980

ctgatcccca tgtgtatcac tggcaaactg tgatggacga caccgtcagt gcgtccgtcg 2040

cgcaggctct cgatgagctg atgctttggg ccgaggactg ccccgaagtc cggcacctcg 2100

tgcacgcgga tttcggctcc aacaatgtcc tgacggacaa tggccgcata acagcggtca 2160

ttgactggag cgaggcgatg ttcggggatt cccaatacga ggtcgccaac atcttcttct 2220

ggaggccgtg gttggcttgt atggagcagc agacgcgcta cttcgagcgg aggcatccgg 2280

agcttgcagg atcgccgcgg ctccgggcgt atatgctccg cattggtctt gaccaactct 2340

atcagagctt ggttgacggc aatttcgatg atgcagcttg ggcgcagggt cgatgcgacg 2400

caatcgtccg atccggagcc gggactgtcg ggcgtacaca aatcgcccgc agaagcgcgg 2460

ccgtctggac cgatggctgt gtagaagtac tcgccgatag tggaaaccga cgccccagca 2520

ctcgtccgag ggcaaaggaa tagagtagat gccgaccgaa caagagctga tttcgagaac 2580

gcctcagcca gcaactcgcg cgagcctagc aaggcaaatg cgagagaacg gccttacgct 2640

tggtggcaca gttctcgtcc acagttcgct aagctcgctc ggctgggtcg cgggagggcc 2700

ggtcgcagtg attcaggccc ttctggattg tgttggtccc cagggcacga ttgtcatgcc 2760

cacgcactcg ggtgatctga ctgatcccgc agattggaga tcgccgcccg tgcctgccga 2820

ttgggtgcag atctcgagat ccggccatta gccatattat tcattggtta tatagcataa 2880

atcaatattg gctattggcc attgcatacg ttgtatccat atcataatat gtacatttat 2940

attggctcat gtccaacatt accgccatgt tgacattgat tattgactag ttattaatag 3000

taatcaatta cggggtcatt agttcatagc ccatatatgg agttccgcgt tacataactt 3060
acggtaaatg gcccgcctgg ctgaccgccc aacgaccccc gcccattgac gtcaataatg 3120
acgtatgttc ccatagtaac gccaataggg actttccatt gacgtcaatg ggtggagtat 3180
ttacggtaaa ctgcccactt ggcagtacat caagtgtatc atatgccaag tacgccccct 3240
attgacgtca atgacggtaa atggcccgcc tggcattatg cccagtacat gaccttatgg 3300
gactttccta cttggcagta catctacgta ttagtcatcg ctattaccat ggtgatgcgg 3360
ttttggcagt acatcaatgg gcgtggatag cggtttgact cacggggatt tccaagtctc 3420
caccccattg acgtcaatgg gagtttgttt tggcaccaaa atcaacggga ctttccaaaa 3480
tgtcgtaaca actccgcccc attgacgcaa atgggcggta ggcatgtacg gtgggaggtc 3540
tatataagca gagctcgttt agtgaaccgt cagatcgcct ggagacgcca tccacgctgt 3600
tttgacctcc atagaagaca ccgggaccga tccagcctcc gcggccccaa gcttatcgat 3660
ttcgaaccca attcgattcg ccaccatggc ctcctccgag aacgtcatca ccgagttcat 3720
gcgcttcaag gtgcgcatgg agggcaccgt gaacggccac gagttcgaga tcgagggcga 3780
gggcgagggc cgcccctacg agggccacaa caccgtgaag ctgaaggtga ccaagggcgg 3840
ccccctgccc ttcgcctggg acatcctgtc cccccagttc cagtacggct ccaaggtgta 3900
cgtgaagcac cccgccgaca tccccgacta caagaagctg tccttccccg agggcttcaa 3960
gtgggagcgc gtgatgaact tcgaggacgg cggcgtggcg accgtgaccc aggactcctc 4020
cctgcaggac ggctgcttca tctacaaggt gaagttcatc ggcgtgaact tcccctccga 4080
cggccccgtg atgcagaaga agaccatggg ctgggaggcc tccaccgagc gcctgtaccc 4140
ccgcgacggc gtgctgaagg gcgagaccca caaggccctg aagctgaagg acggcggcca 4200
ctacctggtg gagttcaagt ccatctacat ggccaagaag cccgtgcagc tgcccggcta 4260
ctactacgtg gacgccaagc tggacatcac ctcccacaac gaggactaca ccatcgtgga 4320
gcagtacgag cgcaccgagg gccgccacca cctgttcctg taaaatcact agtgaattgg 4380
ggtaccgaat tcgatttctg ttcctgttaa tcaacctctg gattacaaaa tttgtgaaag 4440
attgactggt attcttaact atgttgctcc ttttacgcta tgtggatacg ctgctttaat 4500
gcctttgtat catgctattg cttcccgtat ggctttcatt ttctcctcct tgtataaatc 4560
ctggttgctg tctctttatg aggagttgtg gcccgttgtc aggcaacgtg gcgtggtgtg 4620
cactgtgttt gctgacgcaa cccccactgg ttggggcatt gccaccacct gtcagctcct 4680
ttccgggact ttcgctttcc ccctccctat tgccacggcg gaactcatcg ccgcctgcct 4740
tgcccgctgc tggacagggg ctcggctgtt gggcactgac aattccgtgg tgttgtcggg 4800

gaagctgacg tcctttccat ggctgctcgc ctgtgttgcc acctggattc tgcgcgggac 4860

gtccttctgc tacgtccctt cggccctcaa tccagcggac cttccttccc gcggcctgct 4920

gccggctctg cggcctcttc cgcgtcttcg ccttcgccct cagacgagtc ggatctccct 4980

ttgggccgcc tccccgcctg tttcgcctcg ggctcaatca ctagtgaatt cctcgaaaca 5040

tcgataaaat aaaagatttt atttagtctc cagaaaaagg ggggaatgaa agaccccacc 5100

tgtaggtttg gcaagctagc ttaagtaacg ccattttgca aggcatggaa aaatacataa 5160

ctgagaatag agaagttcag atcaaggtca ggaacagatg gaacagctga atatgggcca 5220

aacaggatat ctgtggtaag cagttcctgc cccggctcag ggccaagaac agatggaaca 5280

gctgaatatg ggccaaacag gatatctgtg gtaagcagtt cctgccccgg ctcagggcca 5340

agaacagatg gtccccagat gcggtccagc cctcagcagt ttctagagaa ccatcagatg 5400

tttccagggt gccccaagga cctgaaatga ccctgtgcct tatttgaact aaccaatcag 5460

ttcgcttctc gcttctgttc gcgcgcttct gctccccgag ctcaataaaa gagcccacaa 5520

cccctcactc ggggcgccag tcctccgatt gactgagtcg cccgggtacc cgtgtatcca 5580

ataaaccctc ttgcagttgc atccgacttg tggtctcgct gttccttggg agggtctcct 5640

ctgagtgatt gactacccgt cagcgggggt ctttcatt 5678

<210> 2
<211> 18
<212> DNA
<213> Artificial Sequence

<220>
<223> a primer for RFP probe synthesis

<400> 2
cgccaccatg gcctcctc 18

<210> 3
<211> 18
<212> DNA
<213> Artificial Sequence

<220>
<223> a primer for RFP probe synthesis

<400> 3
caggaacagg tggtggcg 18

<210> 4
<211> 17
<212> DNA
<213> Artificial Sequence

<220>
<223>    sense primer


<400>    4
cgtgaagctg aaggtga                                                    17


<210>    5
<211>    17
<212>    DNA
<213>    Artificial Sequence

<220>
<223>    anti-sense primer


<400>    5
ctcgtactgc tccacga                                                    17


<210>    6
<211>    20
<212>    DNA
<213>    Artificial Sequence

<220>
<223>    sense primer for beta actin


<400>    6
tgccttgaag ttggaaaacg                                                 20


<210>    7
<211>    21
<212>    DNA
<213>    Artificial Sequence

<220>
<223>    anti-sense primer for beta actin


<400>    7
ctggggccta atgttctcac a                                               21

## Description

### TECHNICAL FIELD

The present invention relates to a method for producing cloned transgenic canidae, which has a specific genetic characteristic or from which a specific genetic characteristic has been removed, using transfection of a target gene into somatic cells, gene targeting, and nuclear transfer of the somatic cells transfected with the target gene, and to a cloned transgenic canine produced by said method.

### BACKGROUND ART

The technology for producing transgenic animals is one of technical fields which received the greatest attention over the past twenty years. It is commercially useful and very important in biomedical and biological research. The technology for producing transgenic animals can be industrially used in a very broad range of applications, from production of high-quality livestock products, production of pharmacologically active substances of high added value, production of animals having increased resistance to a variety of pathogenic bacteria and production of disease model animals to gene therapy fields.

The transfer of *RFP* or *GFP* gene as the basic step of the transgenic technology for producing transgenic animals is frequently used for expressing cognate cytoskeletal filaments in living cells, because the *RFP* or *GFP* gene can facilitate labeling of chromosomal proteins and tagging of a specific region of chromosomal DNA, can bind to many cytoplasmic proteins and is non-toxic. In 1994, Chalfie *et al*. observed various molecular biological changes in living cells including porcine embryos using *RFP* obtained from Aequorea victoria as a fluorescent indicator. Since then, enhanced *RFP (ERFP)* has been developed and utilized as a marker gene in several animals.

Methods of transferring a foreign gene into a cell to produce a transgenic animal include pronuclear microinjection suggested by Gordon *et al*. The pronuclear microinjection method comprises injecting a foreign gene directly into the pronucleus of a fertilized oocyte and is frequently applied to experimental animals including mice. However, this method has shortcomings in that, when it is applied to industrial animals, the production yield of transgenic animals is extremely low (0.5% for bovine, 1.5% for pigs, and 2.5% for sheep), and genetic mosaicism occurs in most cases. To overcome these problems, an alternative animal cloning technique was suggested, which employs cells transfected with a foreign gene. The transgenic animal cloning technique can effectively produce transgenic cloned animals by generating 100% transgenic nuclear transfer embryos without genetic mosaicism through nuclear transfer of only cells transfected with a foreign gene, and then transplanting the embryos into surrogate mothers. In addition, in this method, the sex of the transgenic animals can be artificially controlled by previously identifying the sex of somatic cells, thereby maximizing the industrial usefulness of the transgenic animals.

Human organ transplantation is a useful tool for treating organ-related incurable diseases, and cases of organ transplantation have gradually increased for about the past 10 years. Nevertheless the number of people waiting to receive organ transplantation in USA has increased three times for the same period. This is due to an unbalance of supply and demand, meaning shortage of human organs for surgical transplantation. Although organ supply sources are seriously deficient, there is still no satisfactory method capable of solving the problem. Methods to overcome such lack of organs for surgical transplantation include development of artificial organs by medical engineering approaches, and production of transgenic animals.

However, cloning of canines by somatic cell nuclear transfer is very difficult compared to cloning of other animals due to the species-specific reproductive features of the dogs.

During studies on a method for producing a cloned transgenic canine by somatic cell nuclear transfer, the present inventors have produced a cloned dog, which expresses *RFP* gene, by establishing optimal conditions for preparing nuclear donor cells expressing *RFP* gene using gene targeting and transfection and somatic cell cloning, preparing nuclear transfer embryos according to the established conditions, and then transferring the nuclear transfer embryos into a surrogate mother, thereby completing the present invention.

### DISCLOSURE OF INVENTION

It is an object of the present invention to provide a method for producing a cloned transgenic canine, which expresses a target gene, using target gene transfection and somatic cell nuclear transfer, and to provide a cloned canine, which expresses a target gene, produced according to said method.

Another object of the present invention is to provide a method for producing a nuclear transfer embryo for producing said cloned transgenic canine, the nuclear transfer embryo containing a target gene, and to provide a nuclear transfer embryo produced according to said method.

To achieve the above objects, the present invention provides a method for producing a cloned transgenic canine containing a target gene, the method comprising the steps of:
(a) preparing a nuclear donor cell from a somatic cell line collected from a canine, and transfecting and expressing a target gene-containing DNA structure in the nuclear donor cell;
(b) adding to the nuclear donor cell transfected with the target gene a cell cycle synchronization-inducing substance selected from the group consisting of roscovitine, cycloheximide, DMSO, butyrolactone I, aphidicolin, demecolcine, mimosine, colchicine and Hoechst 33342, and culturing the nuclear donor cell;
(c) transferring the cultured nuclear donor cell into an enucleated canine recipient oocyte to generate a transgenic nuclear transfer embryo, and activating the nuclear transfer embryo; and
(d) transferring the activated nuclear transfer embryo into a surrogate mother to produce living offspring.

In the method of the present invention, the nuclear transfer embryo is preferably prepared using the nuclear donor cell which has been synchronized to a specific cycle through a process of adding the cell cycle synchronization-inducing substance such as roscovitine(R-roscovitine) thereto and culturing in step (b). The cell cycle synchronization-inducing substance is preferably added at a concentration of 5-30 µM.

The present invention also provides a cloned transgenic canine containing a target gene, produced by said method.

In another aspect, the present invention provides a method for producing a nuclear transfer embryo for producing a cloned transgenic canine containing a target gene, the method comprising steps (a) to (c) of the above method, and provides a nuclear transfer embryo produced according to said method.

### BRIEF DESCRIPTION OF THE DRAWINGS

FIG. 1 is a schematic view showing a *RFP* gene transfection vector.
FIG. 2 shows fluorescence images of nontransfected fibroblast cells (control group, FIG. 2a), fibroblast cells transfected with REF (FIG. 2b) and embryos cultured after nuclear transfer (FIG. 2c).
FIG. 3 shows the results of Southern blot analysis of the transfected gene *RFP.*
FIG. 4 shows the results of RT-PCR analysis of the mRNA of the transfected gene *RFP.*
FIG. 5 shows a photograph of a 2-month-old cloned transgenic dog containing *RFP* gene and red fluorescence images of the canine skin and canine claw.
FIG. 6 shows fluorescence images indicating the expression of RFP in each tissue of a cloned transgenic canine [a: brain; b: spinal cord; c: testis; d: heart; e: lung; f: kidney; g: liver; h: stomach; and i: small intestine].
FIG. 7 shows FISH analysis results for the mitotic metaphase chromosome of a cloned transgenic canine.

### DETAILED DESCRIPTION OF THE INVENTION AND BEST MODE FOR CARRYING OUT THE INVENTION

Hereinafter, the present invention will be described in detail.

The definition of terms used herein is as follows.

As used herein, the term "target gene" refers to a collection of genes that are expressed in organisms, can perform the respective functions and agree with the intention of the user. The *in vivo* transfection and expression of this target gene is useful for gene therapy for the treatment of a specific disease.

The term "nuclear transfer" as used herein refers to a gene manipulation technique allowing an identical characteristic form and quality acquired by artificially combining an enucleated oocytes with a cell or a cell nucleus.

The term "nuclear transfer embryo" as used herein refers to an embryo injected or fused with a nuclear donor cell.

The term "cloning" as used herein refers to a gene manipulation technique for preparing a new individual unit to have a gene set identical to another individual unit. Particularly, in the present invention, the term "cloning" is used herein to mean that a cell, an embryonic cell, a fetal derived cell, and/or an adult derived cell have a nuclear DNA sequence which is substantially similar or identical to the nuclear DNA sequence of another cell.

The term "nuclear donor cell" as used herein refers to a cell or cell nucleus transferred cell nucleus into a recipient oocyte as a nuclear acceptor.

As used herein, the term "subculture" refers to a method for re-culturing the cells in a new dish after separating the adherent cell which reached confluency from the former culture dish to prevent overgrowth. Specifically, it refers to a method for isolating cells from animals and continuously subjecting the cells to primary culture, secondary culture, tertiary culture, etc., that is, a method for preserving cell lines by periodically replacing with fresh media.

The term "recipient oocyte" as used herein, preferably, refers to an oocyte that receives a nucleus from a nuclear donor cell after removing its original nucleus.

The term "oocyte" as used herein, preferably, refers to a mature oocyte which has reached metaphase II of meiosis.

The term "enucleated oocyte" as used herein refers to an oocyte whose nucleus has been removed.

The term "fusion" as used herein refers to a combination of a nuclear donor cell and a lipid membrane of a recipient oocyte. For example, the lipid membrane may be the plasma membrane or nuclear membrane of a cell. Fusion may occur upon application of an electrical stimulus between a nuclear donor cell and a recipient oocyte when they are placed adjacent to each other or when a nuclear donor cell is placed in the perivitelline space of a recipient oocyte.

The term "activation" as used herein refers to stimulation of a cell to divide, before, during or after nuclear transfer. Preferably, in the present invention, it means stimulation of a cell to divide after nuclear transfer.

The term "living offspring" as used herein means an animal that can survive *ex utero.* Preferably, it is an animal that can survive for one second, one minute, one hour, one day, one week, one month, six months or more than one year. The animal may not require an *in utero* environment for survival.

As used herein, the term "vector" means a DNA molecule which serves as a carrier for stably transporting a base sequence coding for a heterogeneous protein into a host cell. To be useful, a vector must be replicable, readily transformable into host cells, and detectable with a suitable means. By the vector, a foreign gene can be inserted into a specific gene, or the expression of a specific gene can be knocked out. In one embodiment of the present invention, the marker red fluorescent protein (*RFP*) gene was inserted into a transgenic nuclear transfer embryo.

The term "transgenesis" as used herein refers to artificially inserting an exogenous gene into the chromosome of any organism or removing a gene from an organism to change some of the genetic characteristics of the organism. Transgenesis methods are diverse, and typical examples thereof include lipid mediated gene transfer, sperm mediated gene transfer, electroporation, homologous recombination, microinjection and the like.

As used herein, the term "transgenic animal" is also called a "genetically engineered animal" and refers to an animal obtained by artificially inserting an exogenous recombinant gene into the chromosome of an animal or removing a gene from an animal by homologous recombination to change some of the characteristics of the animal. Accordingly, the transgenic animal herein means a bioreactor producing physiologically active substances that human require, a disease model animal genetically displaying a specific disease, and a transgenic animal capable of producing an organ for human transplantation and therapeutic cells.

In the present invention, animals belonging to the family canidae (canines) can be broadly divided into Tribe Canini and Tribe Vulpini, and include dogs, wolves, foxes, jackals, coyotes, dholes and raccoon dogs. Preferably, they include dogs or wolves. The dogs are known to result from the domestication of wild wolves, and thus, wolves and dogs have the same chromosome number and show similarity in gestation period and sex hormone changes (Seal, US et al, Biology Reproduction 1979, 21:1057-1066). In the present invention, the term "animals belonging to the family canidae is also simply abbreviated as "canine".

The present invention relates to a method of producing a cloned transgenic canine, which expresses a target gene, using a combination of gene transfection technology and somatic cell nuclear transfer technology, and to a cloned dog produced according to said method.

More specifically, the present invention relates to a method for producing a cloned transgenic canine, which expresses a target gene, using nuclear transfer of somatic cells transfected with the target gene by gene transfection technology.

The method for producing the cloned transgenic canine expressing the target gene, roughly comprises the steps of:
(a) preparing a nuclear donor cell from a somatic cell line collected from a canine, and transfecting and expressing a target gene-containing DNA structure in the nuclear donor cell;
(b) culturing the nuclear donor cell transfected with the target gene;
(c) transferring the cultured nuclear donor cell into an enucleated canine recipient oocyte to generate a transgenic nuclear transfer embryo, and activating the nuclear transfer embryo; and
(d) transferring the activated nuclear transfer embryo into a surrogate mother to produce living offspring.

In the above method, the target gene-containing DNA structure in step (a) may contain a suitable promoter to regulate the expression of the target gene or may contain a structure similar to the target gene for homologous recombination. Also, step (b) of culturing the nuclear donor cell transfected with the target gene comprises a process of adding a cell cycle synchronization-inducing substance to the nuclear donor cell and then culturing the nuclear donor cell.

The cell cycle synchronization-inducing substance is a substance that temporarily arrests cells in any one phase of the cell cycle consisting of meiotic phase (M), the early phase of DNA synthesis (G1 phase), the DNA synthesis phase (S phase) and the late phase of DNA synthesis (G2 phase), and when the substance is removed, the cell cycle arrested at a specific cycle will progress again. By adding the cell cycle synchronization-inducing substance as described above, the efficiency of production of canine offspring by somatic cell nuclear transfer can be increased.

Examples of the cell cycle synchronization-inducing substance include: roscovitine (Formula 1) as a Cdk (cyclin-dependent kinase) inhibitor blocking the G0/G1 phase; cycloheximide (Formula 2) blocking the G0/G1 phase; dimethyl sulfoxide (DMSO) (Formula 3) blocking the G0/G1 phase; butyrolactone I (Formula 4) as a Cdk inhibitor blocking the G1/S phase; aphidicolin (Formula 5) as an inhibitor of DNA polymerase A_{,}D blocking the early S phase; demecolcine (Formula 6) blocking the M phase in mitotic metaphase; mimosine (Formula 7) as a DNA replication inhibitor blocking the S phase; colchicine (Formula 8) as a microtubule inhibitor blocking the G2/M phase; and Hoechst 33342 (Formula 9) as DNA topoisomerase, and the chemical formula of each of the substances is as follows. Preferred is roscovitine, cycloheximide or DMSO, and the most preferred is roscovitine.

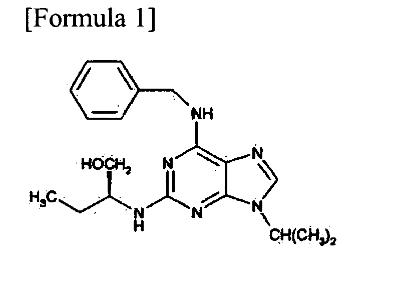

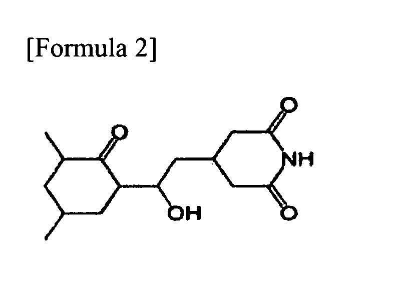

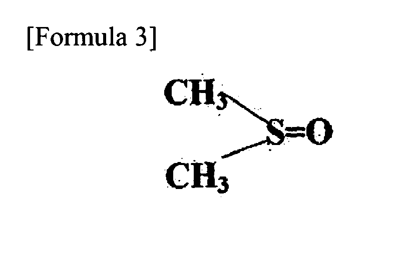

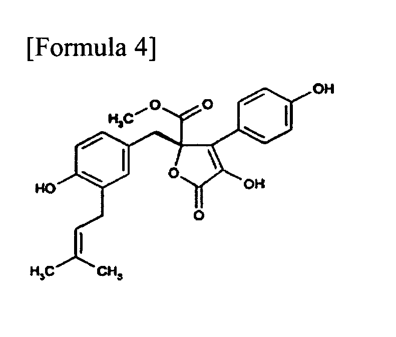

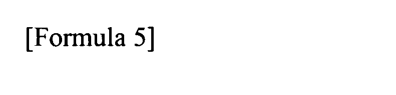

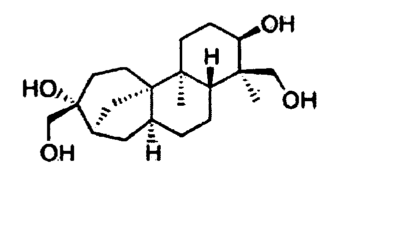

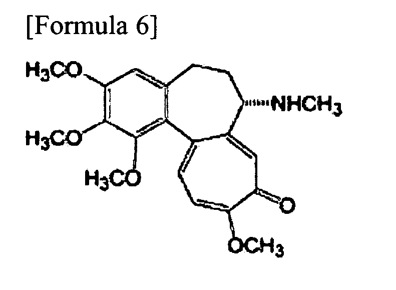

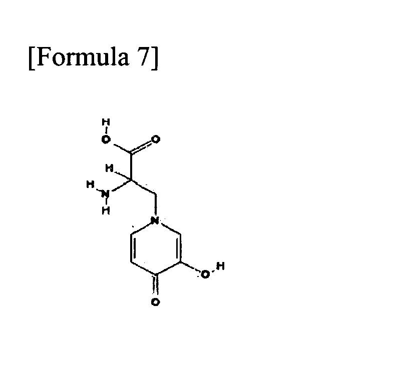

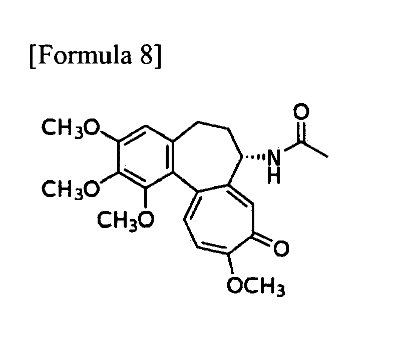

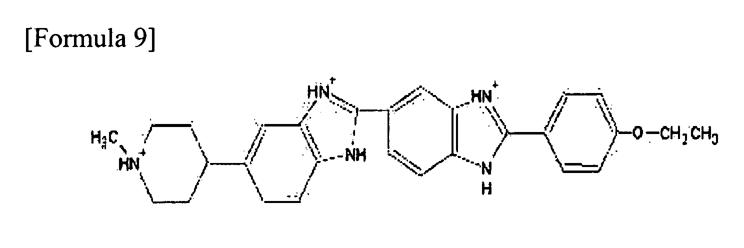

Hereinafter, *RFP* gene is selected as the target gene, and each step of the method for producing a cloned canine in which the target gene is expressed will be described in further detail. It will be apparent to a person of ordinary skill in the art that genes other than *RFP* gene may be used, if necessary.

### Step 1 : Enucleation of recipient oocytes

Generally, the oocytes of mammals (*e*.*g*., cattle, pigs and sheep) are ovulated in mature oocytes, *i*.*e*., metaphase II of meiosis, whereas canine oocytes are ovulated at prophase I of meiosis unlike other animals and matured while staying in the oviduct for 48-72 hours.

Recipient oocytes may be canine immature oocytes, mature oocytes, and oocytes undergoing initial aging, moderate aging and severe aging. Preferably, for use as recipient oocytes, immature oocytes collected from canines can be matured *in vitro,* or oocytes matured *in vivo* can be collected. Because the *in vitro* maturation rate of canine immature oocyte nucleus is very low and the ovulation time and reproductive physiology of canines are different from other animals, it is preferable to collect canine oocytes matured *in vivo* for use as recipient oocytes. More specifically, the collection of mature oocytes from canines is preferably conducted at 48-72 hours and more preferably 72 hours after ovulation induction in the canines.

In this regard, the day of ovulation in canines can be determined by any method known in the art. Examples of the method for determining the day of ovulation include, but are not limited to, vaginal smear tests, the measurement of serum sex hormone levels, and the use of ultrasonographic diagnosis systems. The start of estrus in canines can be confirmed by vulva swelling and serosanguinous discharge and allowance of male mounting.

In one Example of the present invention, vaginal smear test and the analysis of serum progesterone concentration were conducted to determine a day of ovulation. As a result, the day on which nonkeratinized epithelial cells reached more than 80% and serum progesterone concentration initially reached more than about 4.0 ng/mL was regarded as the day of ovulation.

As a method for collecting oocytes matured *in vivo,* a surgical method including anesthetizing an animal followed by laparotomy can be used. More specifically, the collection of oocytes matured *in vivo* can be performed using salpingectomy known in the art. The salpingectomy is a method comprising surgically excising the oviduct, flushing an oocyte collection medium into the oviduct and collecting oocytes from the flushing solution.

In another method, oocytes matured *in vivo* can be collected by inserting a catheter into the fimbriated end of the oviduct, and injecting a flushing solution into the uterotubal junction using an indwelling needle. This method has an advantage in that it does not cause damage to the oviduct, and thus allows an oocyte donor animal to be used for the next estrus.

After the collection of mature oocytes, the haploid nuclei of the oocytes are removed. The enucleation of the oocytes can be performed using any method known in the art (US 4,994,384, US 5,057,420, US 5,945,577, EP 0930009A1, KR 10-0342437; Kanda et al, J. Vet. Med. Sci., 57(4):641-646, 1995; Willadsen, Nature, 320:63-65, 1986, Nagashima et al., Mol. Reprod. Dev. 48:339-343 1997; Nagashima et al., J. Reprod Dev 38:37-78, 1992; Prather et al., Biol. Reprod 41:414-418, 1989, Prather et al., J. Exp. Zool. 255:355-358, 1990; Saito et al., Assis Reprod Tech Andro, 259:257-266, 1992; Terlouw et al., Theriogenology 37:309, 1992).

Preferably, the enucleation of recipient oocytes can be performed by either of the following two methods. One method comprises removing a cumulus cell of a mature recipient oocyte, partially dissecting the zona pellucida of the recipient oocyte using a microneedle by making a slit, and removing the first polar body, nucleus and cytoplasm (in the smallest amount possible) through the slit. Another method comprises removing a cumulus cell of a mature recipient oocyte, staining the oocyte, and removing the first polar body and nucleus of the oocyte using an aspiration pipette. More preferably, for the enucleation of oocytes, the aspiration method is used for oocytes with high viability, whereas the method for forming a slit is used for oocytes with low viability, which is decided by visual examination of the recipient oocytes.

### Step 2: Preparation of nuclear donor cells

In the production of transgenic animals expressing a target gene by somatic cell nuclear transfer technology, nuclear donor cells are required. As nuclear donor cells in the present invention, somatic cells derived from canines are used. Specifically, somatic cells used in the present invention may be canine embryonic cells, fetal cells(fetus cells), juvenile cells, or adult cells, preferably adult derived cells originated from tissue such as the cumulus, skin, oral mucosa, blood, bone marrow, liver, lungs, kidneys, muscles and reproductive organs, etc.

Examples of somatic cells which can be used in the present invention include, but are not limited to, cumulus cells, epithelial cells, fibroblasts, neural cells, keratinocytes, hematopoietic cells, melanocytes, chondrocytes, macropharges, monocytes, muscle cells, B lymphocytes, T lymphocytes, embryonic stem cells, embryonic reproductive cells, fetal cells, placenta cells and embryonic cells. More preferably, somatic cells which can be used in the present invention may be fetal fibroblasts, adult fibroblasts, or cumulus cells. Most preferably, fibroblasts isolated from canine fetuses and adults are used. These cells have advantages in that a large number of the cells can be obtained in the initial isolation stage, culture of the cells is relatively easy, and *in vitro* culture and manipulation of the cells are easy.

The somatic cells which are provided as the nuclear donor cells can be obtained by a method for preparing surgical samples or biopsy samples, and from the samples, single cells cultured under optimized conditions can be obtained using the following method.

Tissue is collected from an animal subject, and cells are isolated from the tissue. Then, the cells are cultured in a tissue culture basal medium, and a cell cycle synchronization- inducing substance is added thereto, followed by the culture of the cells. When the cells completely grow, the cells are collected by treatment with trypsin, and then can be used as nuclear donor cells.

In one embodiment, tissue from an animal to be cloned is aseptically dissected to obtain a surgical sample or a biopsy sample, and the sample is minced, treated with trypsin and then cultured in tissue culture medium. As the tissue culture medium, those known in the art may be used and examples thereof include TCM-199, DMEM (Dulbecco's modified Eagle's medium) and the like. After culturing for 3-4 days in the tissue culture medium, the growth of the cells on a culture dish is confirmed. When the cells completely grow, the cells are treated with trypsin, some of the tissue is frozen and stored in liquid nitrogen for later use, and the remnants are subcultured for use in nuclear transfer. The cells to be continuously cultured for use in nuclear transfer are subcultured in a fresh culture dish, and then treated with trypsin to prepare single cells for use in nuclear transfer.

Somatic cells to be used in gene transfection are grown under optimal conditions, and then prepared into single cells by treatment with an enzyme (trypsin-EDTA). The single cells are subcultured while the culture medium is replaced with a fresh medium at least one day before an experiment and replaced again with a fresh medium 4 hour before the experiment. The cells are cultured until they reach optimal cell density, so that the cells are transfected with the target gene.

### Step 3: Transfection of somatic cell with RFP gene and culture thereof

The nuclear donor cells prepared in step 2 of the present invention may be cells obtained by inserting or manipulating a specific gene in the somatic cells and transforming the chromosome of the cells by a gene transfer method or a gene targeting method. The gene transfer or gene targeting method can be easily practiced by any person skilled in the art because it is known in the art. Examples of the gene targeting method include, but are not limited to, exposure to chemicals after nuclear transfer, injection of inactivated virus, and electroporation, etc.

### 1. Construction of vector for introducing RFP gene and infection

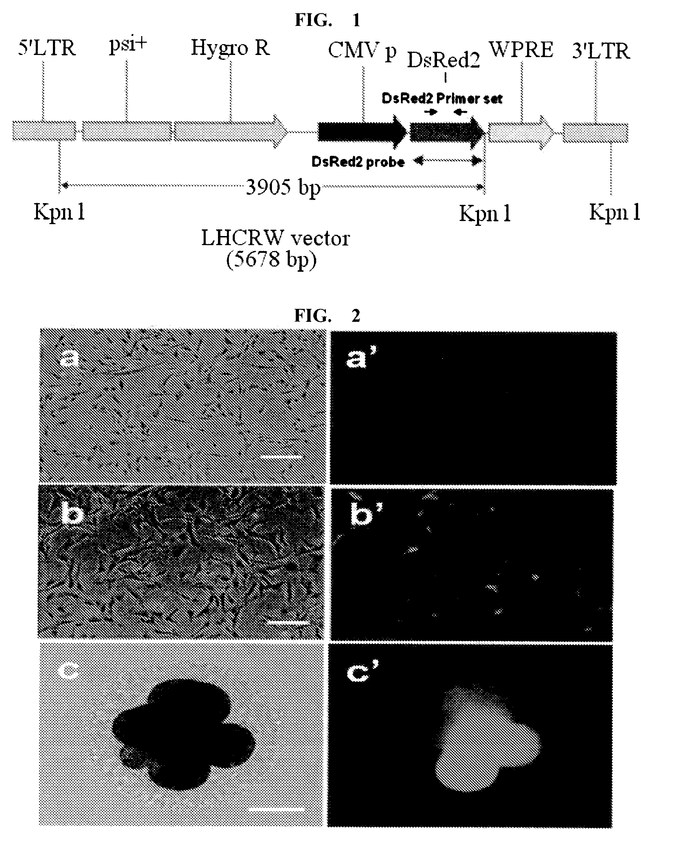

In one embodiment of the present invention, red fluorescent protein (*RFP*) gene can be introduced. To introduce the *RFP* gene, the plasmid pLHCRW (SEQ ID NO.: 1) [5'LTR:1-589, psi sequence:659-1468, HygroR:1510-2544, CMVp:2838-3649, DsRed2:3686-4363, WPRE:4409-5000, 3'LTR:5085-5678] containing a retrovirus vector sequence may be used (FIG. 1).

The pLHCRW plasmid used in the present invention is constructed by replacing the TRE sequence of commercially available pRevTRE (Clontech Mountain View, CA, USA) with a fragment containing the CMV promoter, *DsRed2* gene and WPRE (Woodchuck hepatitis virus posttranscriptional regulatory element) sequences. The CMV promoter and the *DsRed2* gene derived from pLNCX and pDsRed2-C1, respectively (Clontech Mountain View, CA, USA), and the WPRE sequence from Woodchuck hepatitis virus 2 genomic DNA (GenBank Accession No. M11082) can be introduced into the plasmid. Also, retrovirus-producing cells can be constructed following the procedure described by Koo et al. (FASEB, 20: 2251-2260, 2006).

As described above, the vector which is used for introducing the *RFP* gene contains the CMV promoter, but it is apparent to a person of ordinary skill in the art that a vector (DNA structure) containing a gene required for each purpose can contain a promoter suitable for the expression of each target gene. Also, it is apparent to a person skilled in the art that, for homologous recombination, the vector can contain a structure similar to the target gene.

In one embodiment of the present invention, PT67 cells (Clontech Mountain View, CA, USA) are transiently transfected with pLHCRW, and the resultant viruses harvested from the transfected PT 67 cells are applied to GP2-293 cells (Clontech Mountain View, CA, USA). PT67 cells are retrovirus packaging cells that express the gag gene and pol gene of MoMLV (Moloney murine leukemia virus) and Gibbon ape leukemia virus membrane gene, whereas GP2-293 cells express the *gag* and *pol* genes of MoMLV. The LHCRW-infected GP2-293 cells are selected with hygromycin (150 µg/ml) for 2 weeks, and the resultant Hyg^{R} (hygromycin-resistant) cells are transfected with pVSV-G (Clontech) to express VSV-G protein.

Viruses are harvested 48 hours after the transfection. All cells including virus-producing cells are cultured at 37 °C in a 5% CO₂ incubator in Dulbecco modified Eagle medium (DMEM) containing 4.5 g/l glucose (Gibco BRL, Grand Island, NY) supplemented with fetal calf serum (10%) and streptomycin (100 µg/ml). The virus-containing medium harvested from the infected and transfected GP2-293 cells is filtered through a 0.45-µm pore size filter and used to infect canine fibroblast cells. After the infection process, the fibroblasts are selected with hygromycin (150 µg/ml) for 6 days.

In another aspect, the present invention relates to a vector capable of efficiently introducing a target gene, for example, *RFP* gene.

### 2. Selection, proliferation and cryopreservation of nuclear donor cells transfected with RFP gene

The cells transfected with the *RFP* gene through infection with the viral vector are cultured for 6 days in a medium containing hygromycin (150 µg/ml) to select and proliferate only cells expressing the *RFP* gene, and then treated with trypsin enzyme to prepare single cells. Such single cells are observed under a microscope equipped with a UV filter, and only cells having red color are selected and used in nuclear transfer.

When the hyglomycin-resistant gene that is the positive marker used in the *RFP* gene vector is introduced together with the *RFP* gene in cells and expressed in the cells, it produces hyglomycin-resistant proteins. Thus, when the targeted cells are cultured in an antibiotic-containing medium, the cells transfected with the *RFP* gene will survive, and cells other than the transfected cells will be killed by the toxicity of the antibiotic, and thus, after a given period of time, the gene-targeted cells will proliferate in the culture flask.

After the process of selection with the antibiotic has been performed, the cells are returned to normal culture, and a suitable growth factor, an apoptosis inhibitor and the like are added in order to reduce unnecessary loss of the cells during the rapid proliferation and culture of the cells. For efficient preservation of the proliferated and cultured cells, optimal conditions are established, and cryopreservation is carried out in each step.

### 3. Culture of nuclear donor cells transfected with target gene in the presence of cell cycle synchronization-inducing substance

In the present invention, in order to increase the efficiency for producing cloned canines, a cell cycle synchronization-inducing substance is added to the nuclear donor cells transfected with the target gene immediately before nuclear transfer, and the cells are cultured in the presence of the cell cycle synchronization-inducing substance.

Examples of a cell cycle synchronization-inducing substance that can be used in the present invention include: roscovitine (Formula 1) as a Cdk (cyclin-dependent kinase) inhibitor blocking the G0/G1 phase; cycloheximide (Formula 2) blocking the G0/G1 phase; dimethyl sulfoxide (DMSO) (Formula 3) blocking the G0/G1 phase; butyrolactone I (Formula 4) as a Cdk inhibitor blocking the Gl/S phase; aphidicolin (Formula 5) as an inhibitor of DNA polymerase A_{,}D blocking the early S phase; demecolcine (Formula 6) blocking the M phase in mitotic metaphase; mimosine (Formula 7) as a DNA replication inhibitor blocking the S phase; colchicine (Formula 8) as a microtubule inhibitor blocking the G2/M phase; and Hoechst 33342 (Formula 9) as DNA topoisomerase. The chemical formula of each of the substances is as described above. Preferred is roscovitine, cycloheximide or DMSO, and the most preferred is roscovitine.

Immediately before nuclear transfer, roscovitine is added to the cells which are then additionally cultured. The cultured cells are treated with trypsin, harvested and subjected to somatic cell nuclear transfer. The concentration of roscovitine added is preferably 5-30 µM and more preferably 10-20 µM, and the culture time is preferably 18-72 hours and more preferably 24-48 hours.

In one embodiment of the present invention, when the cell concentration reaches about 60% after culture in a fresh culture dish, the cells are treated with 15 µM roscovitine for 18-24 hours, and then treated with trypsin to prepare single cells which are used in nuclear transfer.

### Step 4: Production of nuclear transfer embryos by somatic cell nuclear transfer

In order to produce animals having the same characteristics as those of the nuclear donor cells transfected in the previous step, the present invention employs a cloning technique by somatic cell nuclear transfer (SCNT). Namely, nuclear transfer embryos are produced.

### 1. Microinjection and fusion of nuclear donor cells

The transgenic nuclear donor cells are microinjected into the enucleated oocytes prepared in step 1. Herein, the microinjection is performed by microinjecting the nuclear donor cells between the cytoplasm and zona pellucida of the enucleated oocytes using a transfer pipette.

The enucleated oocytes microinjected with nuclear donor cells are electrically fused with nuclear donor cells using a cell manipulator. The electrical fusion can be performed with direct current or alternating current. Preferably, it can be performed at a voltage of 2.0-6.0 kV/cm, and more preferably, it can be performed 1-3 times at a direct current voltage of 3.0-5.0 kV/cm for 10-30 µs. Most preferably, direct current is applied twice each at a voltage of 3.5-5.0 kV/cm for 15 µs.

The above-described voltage range in the electrical fusion is **characterized in that** it is much higher than a voltage range in general electrical fusion known until now. This range is an optimized condition for electrical fusion and allows the production of cloned dogs with a higher cloning efficiency.

The fusion of the nuclear donor cell to the oocyte by electrical stimulation can be carried out in various fusion media, for example, Zimmerman, sorbitol or mannitol. Preferably, a medium containing mannitol, MgSO₄, Hepes and BSA can be used.

After the enucleated oocytes have been subjected to somatic cell nuclear transfer, and then electrical fusion, the nuclei of the oocytes undergo a remodeling process. As evidence indicating that the remodeling smoothly occurred, premature chromosome condensation (PCC) occurs in the fused embryos at about 1 hour after electrical fusion. It is known that the fused embryos which underwent the premature chromosome condensation are easily developed and reprogrammed after remodeling and activation.

In the case in which nuclear donor cells treated with a cell cycle synchronization-inducing substance such as roscovitine have been transferred, PCC occurs at a rate higher than that in the case in which the nuclear donor cells not treated therewith have been transfected. Also, the continued swelling and recondensation of nuclei occur even after the activation of the fused embryos. Particularly, PCC (premature chromosome condensation), NE (nuclear enlargement) and NS (nuclear swelling), which are processes that nuclei undergo with the passage of time during the normal development thereof, occur in the order of PCC → NE → NS with the passage of time. In cloned embryos obtained by transferring nuclear donor cells treated with a cell cycle synchronization-inducing substance such as roscovitine, PCC occurs at significantly high rate, and thus the nuclear remodeling is more activated.

### 2. Activation of fused nuclear transfer embryos

Activation of fused nuclear transfer embryos is a step of reactivating a cell cycle temporarily arrested in the maturation process. For this purpose, it is necessary to reduce the activity of cell signal transmitters such as MPF, MAP kinase etc., which are factors of cell recycle arrest.

Generally, methods of activating the nuclear transfer embryos include an electrical method and a chemical method. In one embodiment of the present invention, the chemical method is preferably used to activate nuclear transfer embryos.

The chemical method can promote the activation of nuclear transfer embryos according to the present invention more than the electrical method. The chemical methods include a method for treating nuclear transfer embryos with substances such as ethanol, inositol triphosphate, bivalent ions (*e*.*g*., Ca²⁺ or Sr²⁺), microtubule inhibitors (*e*.*g*., cytochalasin B), bivalent ion ionophores (*e*.*g*., Ca²⁺ ionophore ionomycin), protein kinase inhibitors (*e*.*g*., 6-dimethylaminopurine), protein synthesis inhibitors (*e*.*g*., cycloheximide) or phorbol 12-myristate 13 acetate.

Preferably, as the chemical method for the activation of nuclear transfer embryos, a method for treating the nuclear transfer embryos simultaneously or stepwise with calcium ionophore and 6-dimethylaminopurine can be used in the present invention. More preferably, the nuclear transfer embryos are treated with 5-10 µM calcium ionophore at 37-39 °C for 3-6 minutes and then with 1.5 mM-2.5 mM 6-dimethylaminopurine at 37-39 °C for 4-5 hours.

In another aspect, the present invention relates to canine nuclear transfer embryos produced according to the above-described method.

### Step 5: Transfer of nuclear transfer embryos into surrogate mother and production of living offspring

Furthermore, the canine transgenic nuclear transfer embryos may be used to produce cloned canines by transferring them into surrogate mothers to allow living offspring to be born. In the case of canines, nuclear transfer embryos are transferred immediately after activation without *in vitro* culture. The transfer can be performed by any method known in the art, and preferably, a catheter can be used to transfer the cloned embryos.

Surrogate mothers suitable for the transfer of the nuclear transfer embryos and capable of developing the embryos into normal fetuses are selected. The best time for the transfer is determined by monitoring the estrus of canines. Generally, the suitable transfer time is about 48-72 hours from the start of estrus. Thus, a suitable time for transfer of nuclear transfer embryos into a surrogate mother is calculated with consideration of stage of *in vitro* development of the nuclear transfer embryos and the time required therefor. The preferred evaluation of the estrus cycle of a surrogate mother may be based on the concentration of progesterone.

Transfer of the nuclear transfer embryos into a surrogate mother is performed by transferring the embryos into the oviducts of the surrogate mothers by laparotomy. In the transfer of the nuclear transfer embryos into the surrogate mother, the nuclear transfer embryos may preferably be at the 1-cell, 2-cell or 4-cell stage, more preferably, 1-cell stage. For this purpose, the transfer of the nuclear transfer embryos into the surrogate mothers is preferably performed within 4 hours after activation. Also, the nuclear transfer embryos can be cultured in 25 µl microdrops of mSOF covered with mineral oil until surrogate mothers are prepared. As the surrogate mothers, dogs showing an estrus cycle which is the same as or one day later than that of an oocyte donor dog are selected.

4 weeks after transfer of the nuclear transfer embryos, the surrogate mothers are evaluated for pregnancy by ultrasound. After that, the ultrasonic diagnosis is carried out every two weeks to monitor the pregnancy of the surrogate mother and the growth state of fetuses.

If living offspring are not delivered even after the delivery interval has exceeded 30 min, an experienced assistant should help delivery of a surrogate mother. When the expected delivery date is passed, the delivery of offspring is induced by injecting a hormone preparation into the surrogate mother, or by surgical operation such as Caesarean section.

The cloned dog produced according to the method of the present invention has phenotypes similar to those of the nuclear donor cells or the donor, has completely the same genetic properties as those of the nuclear donor cells or the donor, and expresses RFP. In one Example of the present invention, the genetic characteristics of the cloned dog produced according to the method of the present invention were analyzed by microsatellite analysis (see Test Example 2). As a result, it could be seen that the cloned dog according to the present invention had completely the same genetic characteristics as those of the nuclear donor cells or the donor.

### Examples

Hereinafter, the present invention will be described in further detail with reference to examples. It is to be understood, however, that these examples are for illustrative purposes only and are not to be construed to limit the scope of the present invention.

Particularly, although only *RFP* gene was used as a target gene in the present invention, it will be apparent to a person skilled in the art that other target genes may, if necessary, be used and that any promoter suitable for expressing the gene may be selected and used.

### Example 1: Collection of recipient oocytes from dogs

Dogs used as oocyte donors in the experiment were 1-5-year-old mongrel female dogs. The dogs used as oocyte donors were kept according to the standards established by the Seoul National University for Accreditation of Laboratory Animal Care. Ovulation day was determined by performing a vaginal smear test and measuring serum progesterone concentration every day in estrus dogs showing the natural estrus. Also, mature oocytes were surgically retrieved 48-72 hours after ovulation.

In order to measure serum progesterone concentration, 3-5 ml of blood was collected everyday and centrifuged to obtain serum, and the serum was analyzed using a DSL-3900 ACTIVE Progesterone Coated-Tube Radioimmunoassay Kit (Diagnostic Systems Laboratories, Inc., USA). The day on which the progesterone concentration reached 4.0-7.5 ng/ml was considered as the day of ovulation (Hase et al., J. Vet. ed. Sci., 62:243-248, 2000).

To perform the vaginal smear test, smears were obtained daily from the day of the initial sign of proestrus. Smears were collected by inserting a swab into the lips of the vulva and rolled on a slide glass. After staining with a Diff-Quik staining solution (International Chemical Co., Japan), the smears were examined with a microscope; the time at which superficial cells reached more than 80% of the epithelial cells (cornified index, Evans, J.M. et al, Vet. Rec., 7:598-599, 1970) was regarded as the time of ovulation.

The present inventors determined the time of ovulation according to the above-described method, and then retrieved oocytes from donor dogs by laparotomy in the following manner.

First, female dogs as oocyte donors were anesthetized by administering 6 mg/kg of ketamine HCl and 1 mg/kg of xylazine. The anesthesia was maintained by administering isoflurane.

A needle having a rounded front end was inserted into the fimbriated end of the oviduct of the anesthetized dog through the bursal slit. The inserted needle was held in place with suture. Herein, a quick-release device including a 3-cm plastic tube (2 mm diameter) and hemostatic forceps was used. To make the tube of the oviduct well visible, pressure was applied to the oviduct and the surrounding uterus-oviduct junction, and an intravenous catheter (24 gauge) was inserted. Then, Hepes-buffer as an oocyte collection medium shown in Table 1, which contains 10% (v/v) FBS, 2 mM NaHCO₃ and 5 mg/ml BSA (Invitrogen, Carlsbad, CA), was flushed through the catheter to allow oocytes to flow out.

**[Table 1]**

| Ingredient | Amount |
|---|---|
| TCM powder 1 L (Gibco 31100-027) | 9.9g |
| P/S antibiotic | 1 %(penicillin 10000IU, streptomycin 10mg) |
| HEPES buffer | 2.38g |
| FBS | 10%(v/v) |
| NaHCO3 | 0.1680g |
| BSA | 5mg/L |

### Example 2: Enucleation of recipient oocytes

The oocytes obtained in Example 1 were added to the oocyte collection medium of Table 1, and cumulus cells were removed from the oocytes by repeatedly pipetting hyaluronidase (Sigma, USA) in the medium. Then, the oocytes from which cumulus cells have been removed were stained with 5 µg/mL Hoechst 33342 for 5 minutes and observed under an inverted microscope at 200 x magnification so as to select only oocytes having first polar body extruded.

The selected oocytes were enucleated using a micromanipulator (Narishige, Tokyo, Japan) in the above medium (Table 1) supplemented with 5 µg/mL cytochalasin B. Specifically, the oocytes were held with a holding micropipette (about 150 µm diameter), and then the first polar body, adjacent cytoplasm (less than 5%) and oocyte nuclei were removed using an aspiration pipette (about 20 µm diameter). The enucleated oocytes were stored in a TCM-199 medium (Table 2) supplemented with 10% (v/v) FBS.

**[Table 2]**

| TCM-199 Medium | |
|---|---|
| Ingredient | Amount |
| TCM199 liquid | 89ml |
| pyruvic acid | 0.0099g |
| P/S(Antibiotic) | 1ml |
| FBS | 10% |

### Example 3: Preparation of nuclear donor cells

As nuclear donor cells, adult fibroblasts collected from dogs were used. For this purpose, an ear skin biopsy was taken from dogs. Small pieces of the ear tissue fragment were washed three times with DPBS (Dulbecco's Phosphate Buffered Saline) and minced with a surgical blade. The minced tissue was added to 1 mM EDTA-containing DMEM (Dulbecco's modified Eagle's medium) (DMEM Life Technologies, Rockville, MD) and centrifuged at 300 x g for 2 minutes. Then, the cells were seeded into 60 mm plastic culture dishes (Becton Dickinson, Lincoln Park, NJ).

Then, the cells were cultured for 3-4 days in DMEM supplemented with 10% (v/v) FBS, 1 mM glutamine, 25 mM NaHCO₃ and 1% (v/v) minimal essential medium (MEM) nonessential amino acid solution (Invitrogen, CA) at 39 °C in a humidified atmosphere of 5% CO₂ and 95% air.

After the cells have been cultured to confluency, unattached cells were removed, and the remaining attached cells were treated with trypsin in a medium supplemented with 0.1 % trypsin and 0.02% EDTA for 1 minute and were further subcultured at 4-6-day intervals in three fresh culture dishes. Then, the subcultured cells were placed in a freezing medium consisting of 80%(v/v) DMEM, 10%(v/v) DMSO and 10%(v/v) FBS and were stored in liquid nitrogen at -196 °C.

Before performing somatic cell nuclear transfer, the cells were thawed and cultured for 24 hours in medium containing 15 µM roscovitine (*i*.*e*., DMEM + 10% FBS + 15 µM roscovitine). Then, the cells were treated with trypsin for about 2 minutes during somatic cell nuclear transfer and collected from the monolayer.

### Example 4: Transfection of RFP gene and selection of nuclear donor cells transfected with RFP gene

### (1) Construction of gene transfection vector

A *RFP* gene-containing pLHCRW plasmid was prepared in the following manner.

The TRE sequence of commercially available pRevTRE (Clontech Mountain View, CA, USA) was removed from the pRevTRE, and a fragment containing the CMV promoter (derived from pLNCX purchased from Clontech, USA), *DsRed2* gene (derived from pDsRed2-C1 purchased from Clontech, USA) and WPRE (Woodchuck hepatitis virus posttranscriptional regulatory element) sequences (cloned from Woodchuck hepatitis virus 2 genomic DNA) was inserted into the removed position, thus constructing a pLHCRW plasmid.

### (2) Transfection of RFP gene

PT67 cells (Clontech Mountain View, CA, USA) were transiently transfected with pLHCRW, and the resultant viruses harvested from the transfected PT 67 cells were applied to GP2-293 cells (Clontech Mountain View, CA, USA). The LHCRW-infected GP2-293 cells were selected with hygromycin (150 µg/ml) for 2 weeks, and the resultant Hyg^{R} (hygromycin-resistant) cells were transfected with pVSV-G (Clontech Mountain View, CA, USA) to express VSV-G protein.

Viruses packaged with VSG-G were harvested 48 hours after the transfection. All the cells including virus-producing cells were cultured at 37 °C in a 5% CO₂ incubator in DMEM medium containing 4.5 g/l glucose (Gibco BRL, Grand Island, NY), fetal calf serum (10%) and streptomycin (100 µg/ml). The virus-containing medium harvested from the infected and transfected GP2-293 cells was filtered through a 0.45-µm pore size filter and used to infect canine fibroblast cells. After the infection process, the fibroblasts were selected with hygromycin (150 µg/ml) for 6 days.

### (3) Selection and proliferation of nuclear donor cells transfected with RFP gene

*RFP* gene was transfected into donor cells of female dog (BF3) and male dog (BF4) fetal fibroblasts using the retrovirus vector, and stable transfectants (BF3/RFP and BF4/RFP) were isolated from the cells by hygromycin selection. This cell selection using the antibiotic was performed with hygromycin (150 µg/ml) for 6 days at 2-3-day intervals after the gene transfection (FIG. 2b).

After the selection, the cells grew into a colony, treated with trypsin and transferred and cultured on a 96-well plate. After the cells have proliferated, they were transferred and cultured on a 24-well plate, and then cultured on a 12-well plate and a 6-well plate. Among the single cells separated by trypsin treatment, the single cells expressing RFP protein were selected by a UV filter under a microscope and subjected to nuclear transfer.

The cells, which have been transfected with the *RFP* gene, screened with the antibiotic and cultured, were suspended in 15 % fetal bovine serum and 10% medium, and then allowed to stand at 4 °C for 2 hours and at -70 °C for more than 12 hours, followed by cryopreservation at -150 °C.

Before performing somatic cell nuclear transfer, the cells were thawed, and cultured in medium containing 15 µM roscovitine (*i*.*e*., DMEM + 10% FBS + 15 µM roscovitine) for 24 hours. Then, the cells were treated with trypsin for about 2 minutes during somatic cell nuclear transfer and collected from the monolayer.

### Test Example 1: Comparison between group treated with roscovitine and control group

After performing canine somatic cell nuclear transfer into the enucleated oocytes using the microinjection method, the difference in the formation and change of nuclei between a control group not treated with roscovitine and a group treated with roscovitine for 24 hours was examined.

As a result, it could be seen that, in the group treated with roscovitine, premature chromosome condensation (PCC) occurred at a rate higher than that in the control group, and the continued swelling and recondensation of nuclei occurred even after the activation of the fused embryos while showing a significant difference from the control group. Such results indicate that the reconstructed cloned embryos of dogs can be normally remodeled even after the nuclei of canine oocytes have been substituted with the microinjection method and that the treated group develops better than the control group. Various morphological patterns of nuclei resulting from such nuclear remodeling are shown in Table 3 below.

Table 3 below shows the comparison of nuclear remodeling of reconstructed oocytes between the roscovitine-treated group and the control group after transferring somatic cells into enucleated oocytes. PCC, NE and NS indicate phenomena that nuclei undergo with the passage of time during the normal development thereof, and such processes occur in the order of PCC → NE → NS with the passage of time. It was observed that, at 1 hour after electrical fusion, PCC occurred in the treated group at a rate significantly higher than that in the control group. Also, it was observed that, at 4 hours after the activation of the fused embryos, nuclear swelling (NS) occurred more in the treated group than in the control group.

**[Table 3]**

| Time (hpf /hpa) | Treatment | Num.of nuclear trasfer embryo | Num. of reconstructed oocyte | Reconstructed oocyte | | | |
|---|---|---|---|---|---|---|---|
| | | | | IN (%) | PCC (%) | NE (%) | NS (%) |
| 1 hpf | Control group | 32 | 27 | 24(88.8±7.9) | 3(11.1±7.9) | 0 | 0 |
| | Treated group | 39 | 34 | 11(32.3±7.0) | 23(67.6±7.0) | 0 | 0 |
| 4 hpa | Control | 36 | 30 | 0 | 2(6.6±3.7) | 17(56.6±8.4) | 11 (36.6±8.5) |
| | Treated group | 45 | 38 | 0 | 1(2.6±3.3) | 9(23.6±7.5) | 28(73.6±7.5) |

| | | | | | | | |
|---|---|---|---|---|---|---|---|
| (hpf = hour post fusion; hpa = hour post activation; IN = intact nucleus; PCC = premature chromosome condensation; NE = nuclear enlargement; NS = nuclear swelling (P < 0.05).) | | | | | | | |

Next, a group of nuclear donor cells treated with roscovitine according to the inventive method and a control group of nuclear donor cells cultured without treatment with roscovitine were subjected to somatic cell nuclear transfer, and then the pregnancy rate of each of the groups was examined.

As a result, in the control group, 478 embryos transferred with somatic cell nuclei were transferred into 26 surrogate mothers, and among them, 4 animals became pregnant (15.3%, the number of pregnant surrogate mothers/the total number of surrogate mothers). In the group treated with roscovitine, 556 embryos after somatic cell nuclear transfer were transferred into 29 surrogate mothers, and among them, 11 animals succeeded in pregnancy (39.9%, the number of pregnant surrogate mothers/total number of surrogate mothers).

From such results, it could be seen that pregnancy rate was very significantly increased when somatic cell nuclear transfer was performed after treatment with roscovitine.

**[Table 4]**

| Treatment | Num. of transferred embryo | Num. of surrogate mother | Num. of pregnancy | Number of pregnant surrogate mothers/ total number of surrogate mothers | Number of pregnancy/ total number of transferred embryo |
|---|---|---|---|---|---|
| Control | 478 | 26 | 4 | 15.38% | 1.040% |
| Treated with roscovitine | 556 | 29 | 11 | 39.93% | 3.95% |

### Example 5: Somatic cell nuclear transfer

### (1) Microinjection and fusion of nuclear donor cells into enucleated cells

The nuclear donor cells transfected with *RFP* gene, prepared in Example 4, were microinjected into the enucleated oocytes prepared in Example 2. The nuclear donor cells transfected with *RFP* gene were microinjected into the perivitelline space of the enucleated oocytes in the following manner. At the time of microinjecting, the enucleated oocytes were treated with the medium of Table 1 containing 100/mL phytohemagglutinin, and the slit of the enucleated oocytes was held with a holding pipette. Then, a transfer pipette was inserted into the slit, and the single cells isolated from fibroblasts in Example 3 were injected between the cytoplasm and zona pellucida of the enucleated oocytes.

Then, the nuclear donor cell-oocyte couplets which are transfected with *RFP* gene were placed in a fusion medium (containing 0.26 M mannitol, 0.1 mM MgSO₄, 0.5 mM HEPES and 0.05% BSA) and interposed between two parallel electrodes attached to a micromanipulator (Nikon-Narishige, Japan). Then, the fusion of the couplets was induced by electrical stimulation applied twice at a voltage of 4 kV/cm for 15 µs using an electro-cell fusion apparatus (NEPA GENE Co., Chiba, Japan).

The distance between the electrodes was about 180 µm (oocyte diameter). The voltage of the electro-cell fusion apparatus was controlled to 72V, and two pulses were applied for 15 µsec. After 1 hour of electrical stimulation, the fusion of the nuclear donor cells with the oocyte cytoplasm was observed under a stereomicroscope.

After 1 hour of electrical stimulation, the fusion of the nuclear donor cells with the oocyte cytoplasm was observed under a stereomicroscope. The fused embryos were selected and cultured for 1.5-4 hours in TCM-199 (Table 2) supplemented with 10%(v/v) FBS.

### (2) Activation

After the above cell fusion process, the transgenic nuclear transfer embryos were cultured in modified synthetic oviductal fluid (mSOF; Table 5) containing 10 µM ionophore (Sigma) at 39 °C for 4 minutes, thus inducing the activation of the nuclear transfer embryos. Then, the nuclear transfer embryos were washed and further cultured in mSOF supplemented with 1.9 mM 6-dimethylaminopurine for 4 hours. The nuclear transfer embryos were cultured in 25 µl microdrops of mSOF overlaid with mineral oil, before they were transferred into surrogate mothers.

**[Table 5]**

| Ingredient | | | Concentration | Volume |
|---|---|---|---|---|
| NaCl(54.44) 3.100g/ml | 2.900- | | 107.7mM(3.14g) | |
| Kcl(74.55) | | stock-T | 7.2mM | 2ml |
| 0.2669g | | | | |
| KH2PO4 (136.1) 0.0810g | | | 1.2mM | |
| Sod Lactate 0.28ml | | | 3.3mM | |
| Kanamycin 0.0375g | | | | |
| Phenel-Red 0.0050g | | | | |
| NaHC03(84.01) | | Stock-B | 25.1mM | 2ml |
| | 1.0531 | | | |
| | g/50ml | | | |
| NaHCO3(84.01) | | | | |
| | 0.4212 | | | |
| | 4g/20ml | | | |
| Sod.Pyruvate(110.0) 0.0182g/5ml | | Stock-C | 0.3mM | 200 µℓ |
| MgCl26H2O(147.0) 0.0996g/10ml | | Stock-M | 0.5mM | 200 µℓ |
| CaCl22H2O(203.3) 0.2514g/10ml | | Stock-D | 1.71mM | 200 µℓ |
| Glucose(180) 0.27024g/10ml | | | 1.5mM | 200 µℓ |
| Glutamine(146.1) 0.14618g/10ml | | | 1mM | 200 µℓ |
| Citri Acid(192) 0.096g/10ml | | Stock-CA | 0.5mM | 200 µℓ |
| HEPES(238.3) 0.5958g/10ml | | Stock-E | 2.5mM | 200 µℓ |
| EAA(Gibco 11051-018) | | | | 400 µℓ |
| NEAA(Gibco 11140-019) | | | | 200 µℓ |
| ITS(I-3146) | | | | 100 µℓ |
| BSA(fatty acid free) | | | | 0.1600g |
| Hyaluronic Acid | | | 0.5mg/ml | |
| 1N NaOH | | | | |
| D.W. | | | | total 20ml |
| pH 7.2-7.4 / Osmotic pressure 275-285 / Caution with EAA, NEAA which are sensitive to light | | | | |

### Test Example 2: Confirmation of the in vitro development and RFP expression of embryos transferred with somatic cell nucleus

The rate of *in vitro* development of embryos in each of the transgenic SCNT group (SCNT-BF3/REF), the nontransgenic SCNT group (SCNT-BF3) and the parthenogenetic group was comparatively examined (Table 6).

**[Table 6]**

| Embryo type | Num. of embryo | cleaved (%) | 4 cells (%) | 8 cells (%) | 16-32 cells (%) | > Morula(%) | RFP expression (%) |
|---|---|---|---|---|---|---|---|
| Parthen ogenetic group | 42 | 37(87.0±0.04 ) | 27(65.3±0.05) | 22(56.2±0.05) | 11(25.5±0.05) | 1(1.8±0.01) | |
| SCNT-BF3 | 27 | 16(58.7±0.05 ) | 8(29.9±0.04) | 5(17.4±0.06) | 2(6.1±0.07) | | |
| SCNT-BF3/RE F | 54 | 28(53.4±0.07 ) | 15(30.2±0.04) | 9(17.9±0.04) | 3(6.2±0.02) | | 100 |

| | | | | | | | |
|---|---|---|---|---|---|---|---|
| (Average%± SEM; p<0.05) | | | | | | | |

Although the total rate of development of the SCNT embryos was significantly lower than that of the parthenogenetic group, the transgenic group and the nontransgenic group showed similar embryonic development rates 53.4±0.07 vs. 58.7±0.05% at the 2-cell stage, 30.2±0.04 vs. 29.9±0.04% at the 4-cell stage, 17.9±0.04 vs. 17.4±0.06% at the 8-cell stage, and 6.2±0.02 vs. 6.1±0.07% at the 16-32 cell stages. The transgenic group showed red fluorescence at the 2-, 4-, 8-16-cell stages without mosaicism (FIG. 2c), whereas the control group showed no red fluorescence.

### Example 6: Embryo transfer into surrogate mothers and production of cloned dogs

The nuclear transfer embryos from Example 5 were surgically transferred into the oviduct of estrus synchronized surrogate mothers. The transfer was carried out within 4 hours after the activation of the nuclear transfer embryos. As the surrogate mothers, dogs were used, which were disease-free, showed the repetition of the normal estrus cycle and had a normal uterine condition. A total of 344 embryos (287 BF3/RFP embryos and 57 BF4/RFP embryos) were transferred into the oviducts of 20 surrogate mothers. Specifically, the BF3/RFP embryos were transferred into 16 surrogate mothers, and the BF4/RFP embryos were transferred into 4 surrogate mothers.

For this purpose, the surrogate mothers were anesthetized by injection with 6 mg/kg ketamine HCl and 1 mg/kg xylazine, and maintained at the inhalation-anesthetized state using 2% isoflurane. Operation area of the anesthetized dogs was aseptically operated and incised on the center of the abdomen according to general laparotomy so as to expose the oviduct. The abdominal cavity was stimulated by hand to draw the ovary, the oviduct and the uterus to the incision. The mesovarium of the drawn ovary was carefully handled to recognize the opening of the oviduct, and a 3.5F Tom cat catheter (Sherwood, St. Louis, MO) equipped with a 1.0 ml tuberculin syringe (Latex free, Becton Dickinson & CO. Franklin lakes, NJ 07417) was inserted into the oviduct to secure a sufficient space in the front of the catheter. Then, the nuclear transfer embryos were injected into the oviduct through the catheter. Whether the nuclear transfer embryos were successfully injected was observed under a microscope. The abdominal suture was performed with an absorbable suture material, and then, skin suture was performed. To prevent post-surgery infection, a broad range of antibiotic was injected for 3 days.

At 23 days after transferring the nuclear transfer embryos into the surrogate mothers, pregnancies were detected using a SONOACE 9900 ultrasound scanner (Medison Co. LTD, Seoul, Korea) equipped with a 7.0 MHZ linear-array probe. Specifically, at 23 days after the embryo transfer, an embyonic sac was detected in each surrogate mother by ultrasound, and no fetal death or miscarriage was detected. Ultrasonic examination carried out about 25 days after the embryo transfer showed that 7 animals among the surrogate mothers became pregnant (5 animals: BF3/RFP, and 2 animals: BF4/RFP) (Table 7). 60 days after the embryo transfer, 4 female animals (named "R1" to "R3" and "R5") and 2 male animals (named "R6" and "R7") were born by inducing natural delivery or performing Cesarean section.

**[Table 7]**

| Nuclear donor cells | BF3/ RFP | BF4/RFP | Total |
|---|---|---|---|
| Sex of donor | Female | Male | |
| *In vivo* oocytes | 434 | 74 | 508 |
| Enucleated oocytes | 405 | 66 | 471 |
| Celltransferred oocytes | 396 | 66 | 462 |
| Fusion attempted | 384 | 66 | 450 |
| Fused embryos (%) | 315(82) | 57(86.4) | 372(82.7) |
| Embryos transferred to recipients | 287 | 57 | 344 |
| Recipients | 16 | 4 | 20 |
| Pregnancies / recipients (%) | 5(31.3) | 2(50.0) | 7(35.0) |
| Births / embryos transferred (%) | 4(1.4) | 2(3.5) | 6(1.7) |

### Test Example 3: Examination of characteristics of cloned dogs produced according to method of the present invention

The characteristics of the pups produced by nuclear transfer are summarized in Table 8 below. The transgenic cloned pups were genetically identical to the respective donor cells.

**[Table 8]**

| ID | Donor cell type | Gestation (days) | Birth weight(Kg) | Karyotype | status | Comments |
|---|---|---|---|---|---|---|
| R1 | BF3/RFP | 61 | 310 | 76 + XX | Live | C-sec |
| R2 | BF3/RFP | 59 | 270 | 76 + XX | Live | C-sec |
| R3 | BF3/RFP | 60 | 240 | 76 + XX | Live | Natural delivery |
| R5 | BF3/RFP | 60 | 260 | 76 + XX | Live | Natural delivery |
| R6 | BF4/RFP | 61 | 330 | 76 + XX | Dead (2 mo) | C-sec |
| R7 | BF4/RFP | 60 | 260 | 76+XX | Live | Natural delivery |

Also, it was shown that the mtDNAs of the transgenic pups were all derived from the oocytes of the donor dogs (data not shown).

### Test Example 4: In vivo confirmation of expression of RFP gene in cloned dogs produced according to method of the present invention

The genetic analysis of each of the transgenic living offspring produced in Example 6 was performed by a visual method and a molecular biological method. The cloned dogs expressing RFP were observed visually, and RFP expression and gene transfection therein were analyzed by Southern blot using tissue and by a UV filter of a microscope.

### (1) Southern blot analysis and RT-PCR analysis

By molecular biological analysis, the genomic DNAs of the living offspring were analyzed by southern analysis. In the southern blot analysis, 5'-CGCCACCATGGCCTCCTC-3' (SEQ ID NO.: 2) and 5'-CAGGAACAGGTGGTGGCG-3' (SEQ ID NO.: 3) corresponding to positions 606-623 and positions 1270-1287, respectively, of a pDsRed2-C1 (Clontech, Mountain View, CA, USA) nucleotide sequence, were used as primers for the synthesis of a *RFP* gene probe, pDsRed2-C1. The probe (3905 bp) was synthesized using the PCR DIG probe synthesis kit (Roche, Mannheim, Germany), purified by agarose gel electrophoresis, and labeled with digoxigenin before hybridization.

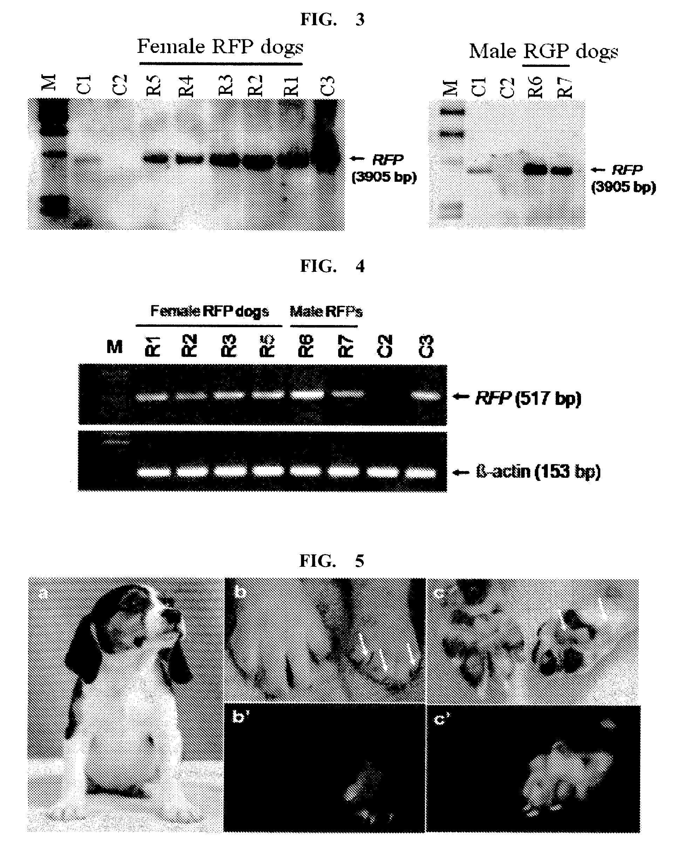

As a result, labeled DNA on the positively charged nylon membrane was detected by a DIG luminescent detection kit (Roche, Mannheim, Germany) (FIG. 3).

To confirm the expression at transcriptional level of *RFP* gene, RT-PCR was performed for the cloned dogs. The total RNA was treated with DNase I and reverse-transcribed using the first-strand cDNA synthesis kit (Amersham Pharmacia Biotech, Oakville, ON, Canada). In RT-PCR, the *RFP* cDNA was amplified using the forward primer RFP-F (5'-CGTGAAGCTGAAGGTGA-3' (SEQ ID NO.: 4)) and the reverse primer RFP-R (5'-CTCGTACTGCTCCACGA-3' (SEQ ID NO.: 5)), thus obtaining an amplified product (517 bp). A β-actin forward primer(5'-TGCCTTGAAGTTGGAAAACG-3' (SEQ ID NO.: 6)) and a β- actin reverse primer (5'-CTGGGGCCTAATGTTCTCACA-3' (SEQ ID NO.: 7)) were used to modulate the transcription of β-actin, thus obtaining a β-actin transcription product (153 bp).

The results of RT-PCR are shown in FIG. 4. Namely, the expression at transcription level of *RFP* gene was confirmed.

### (2) Detection of development of red fluorescence

The detection of red fluorescence was performed using a Leica inverted microscope equipped with a Texas red filter set and a Leica upright stereomicroscope equipped with a DsRed filter set. The development of red fluorescence appears with emission at 540±0 nm and is detected by an emission filter with a maximal transmittance wavelength of 600 ± 5 nm.

Offspring expressing DsRed were identified using a GFsP-5 head light source (Biochemical Laboratory Services, Budapest, Hungary).

As a result, as can be seen in FIG. 5, red fluorescence was detected in the transgenic cloned dogs, but no red fluorescence was detected in the nontransgenic dogs as the control group (FIGS. 5b and 5c).

Also, the skin and claw of the cloned transgenic dogs were irradiated with light from a 70W surgery light equipped with an excitation filter (HQ 540/40; Chroma Technology Corp., Rockingham, VT, USA), red fluorescence emission from the skin and claw was detected using a Samsung digital camera (GX10, Republic of Korea) equipped with emission filters (HQ 600/50; Chroma Technology Corp., Rockingham, VT, USA) that were attached proximal to the camera lens (FIGS. 5b' and 5c').

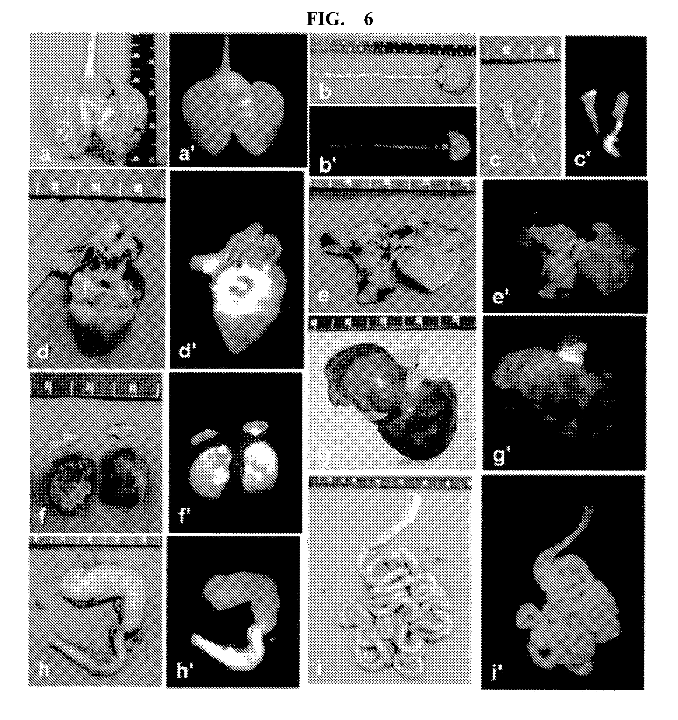

Also, as can be seen in FIG. 6, the cloned transgenic dogs containing *RFP* gene expressed *RFP* gene in the tissues, including the brain, heart, liver, kidney, testis, lung, muscle, small intestine, thymus, spleen, skin and stomach. On the contrary, in the control cloned dogs containing no *RFP* gene, no fluorescent substance was detected.

Meanwhile, fibroblasts were collected from the transgenic living offspring, and the expression of RFP in the collected fibroblasts was observed using an inverted microscope equipped with a RFP specific filter (EX 510-560 nm, BA 590 nm) and analyzed with FACS (fluorescence activated cell sorter). For this purpose, the fibroblasts were washed with PBS, treated with trypsin to detach the attached cells, and then fixed with 70% ethanol at 4 °C for 10 minutes. The cells were analyzed using a FACS Caliber (Becton-Dickinson, NY, USA). As a result, the expression of RFP in the fibroblasts of the transgenic cloned dogs was confirmed (data not shown).

### (3) FISH (fluorescence in situ hybridization) analysis

The insertion of *RFP* gene was confirmed by fluorescence *in situ* hybridization (FISH) analysis.

For FISH analysis, a RFP-specific probe was labeled with biotin using a biotin nick translation mix kit (Roche, Mannheim, Germany), and 100 ng of the labeled probe was mixed with a mixture of salmon sperm DNA and Hyb [50% formamide, 10% dextran sulfate (Sigma), 2x SSC], and then denatured at 75 °C for 5 minutes. The denatured chromosomes were incubated overnight at 37 °C. Then, the chromosomes were washed with 50% formamide at 2x SSC at 45 °C for 30 minutes, additionally washed at 2x SSC for 5 minutes, and then rinsed with 4xSSC/0.1% Tween 20. Then, the chromosomes were treated with fluorescein avidin DCS (Vector Laboratories Inc., Burlingame, USA), incubated at 37 °C for 20 minutes, and then washed with 4x SSC. Next, they were treated with immunopure biotinylated goat anti-avidine (Pierce, Rockford, USA) and incubated again at 37 °C for 20 minutes. After washing, they were treated with fluorescein avidin DCS. Then, the slides were washed and counterstained with 4',6-diamidino-2-phenylindole (Vector Laboratories Inc.). FISH images were examined using Leica DMRXA2 (Leica Microsystems, Wetzlar, Germany), captured by a CoolSNAP cf digital camera (Roper Scientific photometrics, Tucson, USA), and analyzed by Leica CW4000 (Leica Microsystems, Wetzlar, Germany).

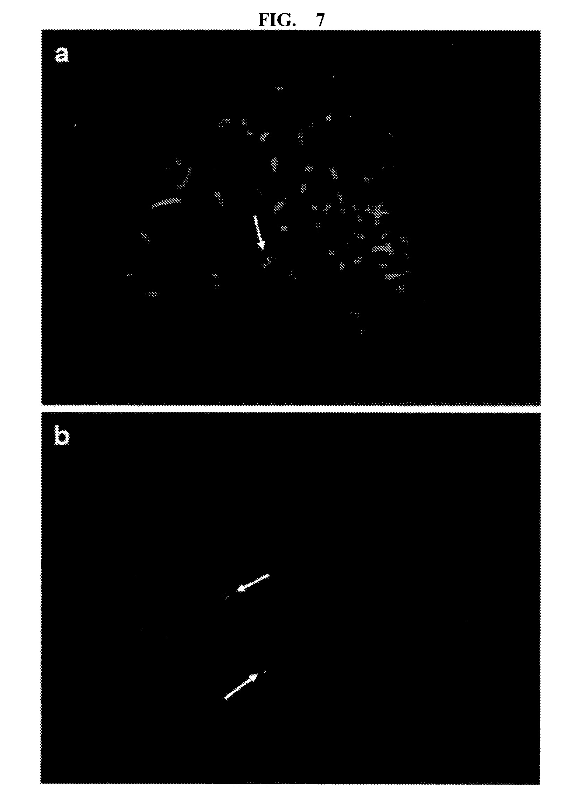

As a result, as can be seen in FIG. 7, the insertion of *RFP* gene in the mitotic metaphase chromosomes occurred at one point (FIG. 7a) or two points (FIG. 7b), and in the case in which the insertion occurred at two points, the insertion occurred on different chromosomes.

The above results suggest that, according to the method of the present invention, a cloned transgenic canine containing a target gene can be produced.

While the present invention has been described with reference to the particular illustrative embodiments, it is obvious for those skilled in the art that these illustrative embodiments are preferable examples, not to be restricted by the embodiments. Therefore, the practical range of the present invention is defined as appended claims and equivalent thereof.

### INDUSTRIAL APPLICABILITY

As described above, the present invention provides a cloned transgenic canine, which expresses a target gene, by introducing the target gene into somatic cells, and then subjecting the somatic cells to nuclear transfer cloning. Accordingly, the present invention can produce human and animal disease models, or organs or therapeutic cells that can be transplanted into the human body without hyperacute immune rejection.

## Claims

1. A method for producing a cloned transgenic canine containing a target gene, the method comprising the steps of:
(a) preparing a nuclear donor cell from a somatic cell line collected from a canine, and transfecting and expressing a target gene-containing DNA structure in the nuclear donor cell;
(b) adding to the nuclear donor cell transfected with the target gene a cell cycle synchronization-inducing substance selected from the group consisting of roscovitine, cycloheximide, DMSO, butyrolactone I, aphidicolin, demecolcine, mimosine, colchicine and Hoechst 33342, and culturing the nuclear donor cell;
(c) transferring the cultured nuclear donor cell into an enucleated canine recipient oocyte to generate a transgenic nuclear transfer embryo, and activating the nuclear transfer embryo; and
(d) transferring the activated nuclear transfer embryo into a surrogate mother to produce living offspring.

2. The method according to claim 1, the somatic cell in step (a) is one selected from the group consisting of cumulus cell, epithelial cell, fibroblast, neural cell, keratinocyte, hematopoietic cell, melanocyte, chondrocyte, macropharge, monocyte, muscle cell, B lymphocyte, T lymphocyte, embryonic stem cell, embryonic reproductive cell, fetal cell, placenta cell and embryonic cell.

3. The method according to claim 2, the somatic cell is fibroblast or cumulus cell.

4. The method according to claim 1, wherein the target gene-containing DNA structure in step (a) additionally contains a promoter suitable to express the target gene.

5. The method according to claim 1, wherein the cell cycle synchronization-inducing substance in step (b) is roscovitine.

6. The method according to claim 1, wherein the cell cycle synchronization-inducing substance in step (b) is added at a concentration of 5-30 µM.

7. A cloned transgenic canine containing a target gene, produced by the method according to claim 1.

8. A method for producing a nuclear transfer embryo containing a target gene, the method comprising the steps of:
(a) preparing a nuclear donor cell from a somatic cell line collected from a dog, and transfecting and expressing a target gene-containing DNA structure in the nuclear donor cell;
(b) adding to the nuclear donor cell transfected with the target gene a cell cycle synchronization-inducing substance selected from the group consisting of roscovitine, cycloheximide, DMSO, butyrolactone I, aphidicolin, demecolcine, mimosine, colchicine and Hoechst 33342, and culturing the nuclear donor cell; and
(c) transferring and fusing the cultured nuclear donor cell into an enucleated canine recipient oocyte.

9. A nuclear transfer embryo for producing a cloned transgenic canine containing a target gene, produced according to claim 8.
